(19) Europäisches Patentamt
European Patent Office
Office européen des brevets

(11) Veröffentlichungsnummer: **0 019 112**
**B1**

(12) # EUROPÄISCHE PATENTSCHRIFT

(45) Veröffentlichungstag der Patentschrift: **17.11.82**

(51) Int. Cl.³: **C 07 D 319/06**

(21) Anmeldenummer: **80102184.1**

(22) Anmeldetag: **23.04.80**

(54) **Verfahren zur Herstellung von cyclischen Acetalen von trans-4-Chlor-3-methyl-2-buten-1-al sowie von trans-3-Methyl-2-buten-1,4-dial-1-monoacetalen.**

(30) Priorität: **28.04.79 DE 2917413**

(43) Veröffentlichungstag der Anmeldung: **26.11.80 Patentblatt 80/24**

(45) Bekanntmachung des Hinweises auf die Patenterteilung: **17.11.82 Patentblatt 82/46**

(84) Benannte Vertragsstaaten: **BE CH DE FR GB LI NL**

(56) Entgegenhaltungen:
**DE-A-2 225 612**
**DE-A-2 357 752**
**DE-A-2 513 999**

(73) Patentinhaber: **BASF Aktiengesellschaft, Carl-Bosch-Strasse 38, D-6700 Ludwigshafen (DE)**

(72) Erfinder: **Jaedicke, Hagen, Dr., Budapester Strasse 49, D-6700 Ludwigshafen (DE)**
Erfinder: **Paust, Joachim, Dr., Ringstrasse 3, D-6701 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von cyclischen Acetalen von trans-4-Chlor-3-methyl-2-buten-1-al sowie von trans-3-Methyl-2-buten-1,4-dial-1-monoacetalen

Die Erfindung betrifft ein Verfahren zur Herstellung von 6-Ring-Acetalen von trans-4-Chlor-3-methyl-2-buten-1-al durch Umsetzen der entsprechenden Acetale des 3-Methyl-2-buten-1-als (Prenal) mit Sulfurylchlorid sowie deren anschließende Oxidation zur Herstellung der für Terpensynthesen begehrten trans-3-Methyl-2-buten-1,4-dial-1-monoacetale (3-Methyl-fumardialdehyd-1-monoacetalen).

Die trans-3-Methyl-2-buten-1,4-dial-1-monoacetale haben große Bedeutung, da es mit ihrer Hilfe möglich ist, successive Wittig-Reaktionen zu zahllosen Verbindungen mit biologischer und pharmakologischer Bedeutung durchzuführen. Beispielsweise kann man durch Umsetzen von trans-3-Methyl-2-buten-1,4-dial-1-acetalen mit dem Ylid von $\beta$-Ionylidenäthyltriphenylphosphoniumsalzen und anschließende Hydrolyse auf einfache Weise das begehrte Retinal erhalten. Retinal besitzt die gleiche Wirksamkeit wie Vitamin A. Aus Retinal kann man außerdem durch einfache Wittig-Reaktion mit dem aus Retinol leicht erhältlichen Retinyltriphenylphosphoniumsalz auf sehr wirtschaftliche Weise $\beta$-Carotin herstellen.

Für die Herstellung der trans-3-Methyl-2-buten-1,4-dial-1-acetale sind verschiedene Verfahren bekannt.

So ist z. B. in der DE-A-2 225 612 ihre Herstellung durch Oxidation von cyclischen 3-Methyl-2-buten-4-ol-1-al-acetalen mit schwefelsaurer Chromsäurelösung in Aceton beschrieben. Dieses Verfahren verläuft mit relativ guten Ausbeuten. Jedoch ist seine technische Realisierung durch die Verwendung von Chromsäure als Oxidationsmittel und die durch die Giftigkeit von Chromverbindungen bedingten großen Abwasserprobleme sehr erschwert.

Aus der DE-A-2 357 752 ist es bekannt, die begehrten trans-3-Methyl-2-buten-1,4-dial-1-acetale durch Oxidation der entsprechenden 3-Methyl-2-buten-1-al-acetale mit Selenoxid in bestimmten Lösungsmitteln herzustellen. Bei diesem Verfahren sind die erzielten Ausbeuten unbefriedigend. Außerdem ist die technische Durchführung des Verfahrens durch die große Toxizität des Selens sehr problematisch.

Weiterhin ist aus der DE-A-2 513 999 bekannt, die trans-3-Methyl-2-buten-1,4-dial-1-acetale ausgehend von Crotonaldehydacetalen durch Ozonolyse mit anschließender reduktiver Aufarbeitung, Grignard-Vinylierung und Acetylierung der erhaltenen Glyoxalmonoacetale sowie Hydroformylierung der erhaltenen neuen 2-Acetoxy-3-en-1-al-acetale und Eliminierung von Essigsäure herzustellen. Jedoch bringen die zur Durchführung dieses Verfahrens notwendigen vielen und teilweise aufwendigen Verfahrensschritte relativ hohe Herstellkosten mit sich.

Es war daher die Aufgabe der Erfindung ein Verfahren zu entwickeln, das es ermöglicht, die begehrten trans-3-Methyl-2-buten-1,4-dial-1-acetale auf technisch besonders einfache Weise, d. h. mit möglichst geringem Arbeitsaufwand herzustellen.

Es wurde nun gefunden, daß sich die gut zugänglichen 6-Ringacetale von 3-Methyl-2-buten-1-al (Prenal) unter ganz bestimmten Bedingungen mittels Sulfurylchlorid mit großer Selektivität in die trans-4-Chlor-3-methyl-2-buten-1-al-acetale überführen lassen, welche unter milden Oxidationsbedingungen mit guten Ausbeuten zu den gewünschten trans-3-Methyl-2-buten-1,4-dial-1-monoacetalen oxidiert werden können.

Gegenstand der Erfindung ist dementsprechend ein Verfahren zur Herstellung von Acetalen der allgemeinen Formel I

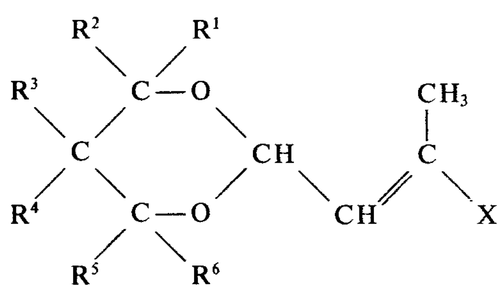

(I)

in der $R^1$ bis $R^6$ für $-H$, $-CH_3$ oder $-C_2H_5$, vorzugsweise $-H$ oder $-CH_3$ stehen, wobei vorzugsweise nur 1 bis 4 der Reste $R^1$ bis $R^6$ für $-CH_3$ und die restlichen für $-H$ stehen und X für $-CH_2Cl$ oder

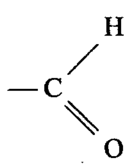

steht, das dadurch gekennzeichnet ist, daß man

A) in die Lösung des entsprechenden Acetals von 3-Methyl-2-buten-1-al der Formel II

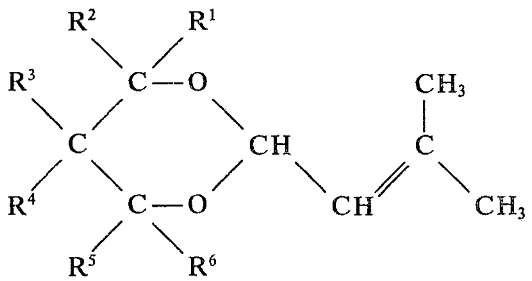

(II)

in einem Halogenkohlenwasserstoff vom Siedepunkt 50 bis 120°C bei Temperaturen von 50 bis 120°C, vorzugsweise 80 bis 90°C, molare bis leicht überschüssig molare Mengen von Sulfurylchlorid langsam und in dem Maße wie das Sulfurylchlorid im Reaktionsgemisch verbraucht wird, einträgt, und

B) für den Fall, daß X für

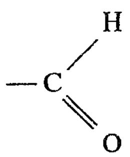

steht, das erhaltene 4-Chlor-3-methyl-but-2-en-1-al-acetal unter milden Reaktionsbedingungen oxidiert.

Mit besonderem Vorteil verläuft das erfindungsgemäße Verfahren, wenn man im Reaktionsschritt A das Sulfurylchlorid in eine siedende Lösung des entsprechenden Acetals der Formel II in Trichloräthylen einträgt.

Die besten Selektivitäten an 4-Chlor-3-methyl-2-buten-1-al-acetalen erzielt man bei dem erfindungsgemäßen Verfahren dann, wenn man im Reaktionsschritt A pro Mol des Acetals etwa 1,1 Mol Sulfurylchlorid innerhalb von 1 bis 2 Stunden in die Lösung des Acetals einträgt.

Daß sich die 6-Ring-Acetale von 3-Methyl-2-buten-1-al unter den erfindungsgemäßen Bedingungen mit großer Selektivität in die gewünschten 4-Chlor-3-methyl-but-2-en-1-al-1-acetale überführen lassen, ist aus den im folgenden erörterten Gründen sehr überraschend.

In einer Übersichtsarbeit in Houben Weyl »Methoden der organischen Chemie«, Band 5/3 über die Verwendung von Sulfurylchlorid als Chlorierungsmittel heißt es beispielsweise, daß die Addition von Chlor an ungesättigte Verbindungen mit Sulfurylchlorid leicht unter Bildung gesättigter Dichlorderivate und $SO_2$ eintritt (vgl. loc. cit. S. 874). Demgemäß hätte man erwarten müssen, daß die 3-Methyl-2-buten-1-al-acetale bei Umsetzen mit $SO_2Cl_2$ im wesentlichen 2,3-Dichlor-butan-1-al-acetale bilden, was ja auch bei Verwendung von Kohlenwasserstoffen wie Petroläther und Cyclohexan als Lösungsmittel quantitativ der Fall ist. Weiterhin heißt es in der genannten Arbeit, daß Sulfurylchlorid 2 Arten von Additionsreaktionen liefern kann: es vermag 2 Chloratome an die olefinische Doppelbindung anzulagern oder kann mit seiner ganzen Molekel unter Bildung von Chloralkansulfonsäurechloriden reagieren (vgl. loc. cit. S. 875 oben). Auch diese Aussage hätte nicht die erfindungsgemäße Umsetzung erwarten lassen. Weiterhin heißt es in der genannten Arbeit, daß bei der Chlorierung mit Sulfurylchlorid wie bei der Chlorierung mit elementarem Chlor sekundärer Wasserstoff leichter reagiert als primärer und tertiärer leichter als sekundärer (vgl. loc. cit. S. 877 Absatz 2). Dementsprechend hätte man erwarten müssen, daß im wesentlichen 2-Chlor-3-methyl-3-buten-1-al-acetale gebildet würden.

Bemerkenswert ist noch, daß diese Chlorierung mit $SO_2Cl_2$ nur mit 6-Ring-acetalen gelingt. Bei Verwendung von 5-Ring-acetalen des Prenals oder von einfachen Acetalen, wie dem Dimethylacetal, werden die Acetale von $SO_2Cl_2$ zerstört. Bei Versuchen $Cl_2$ als Chlorierungsmittel zu verwenden sowohl die 5-Ring-Acetale als auch die 6-Ring-acetale zerstört.

Weiterhin sollte erwähnt werden, daß bei der Umsetzung von Tiglinaldehyd-6-Ring-acetalen, d. h. also von Verbindungen, die sich von den Verbindungen der Formel II nur durch die Stellung der Methylgruppe unterscheiden, auch unter den erfindungsgemäßen Bedingungen ausschließlich das vicinale Dichlorid gebildet wird.

Die als Ausgangsstoffe für das erfindungsgemäße Verfahren benötigten Acetale erhält man durch

Acetalisierung von Prenal mit den definitionsgemäßen 1,3-Diolen. Prenal seinerseits ist ein im Handel erhältliches Produkt. Es kann beispielsweise durch Umsetzen von Isobutylen und Formaldehyd, Isomerisierung des dabei erhaltenen 2-Methyl-1-buten-4-ols und anschließende Dehydrierung des dabei erhaltenen 3-Methyl-2-buten-1-ols (vgl. DE-A-2 041 976) erhalten werden.

Als geeignete Ausgangsstoffe seien beispielsweise die Acetale von 3-Methyl-2-buten-1-al mit Butan-1,3-diol, Pentan-2,4-diol, Propan-1,3-diol und insbesondere Neopentylglykol (2,2-Dimethyl-propan-1,3-diol) genannt.

Sulfurylchlorid ist eine im Handel erhältliche Verbindung.

Als Lösungsmittel kommen für die erfindungsgemäße Umsetzung Halogenkohlenwasserstoffe in Betracht, die zwischen etwa 50 und 120°C sieden. Genannt seien beispielsweise Dichloräthylen, Trichloräthylen, Tetrachloräthylen, Chloroform und Chlorbenzol. Mit ganz besonders guter Selektivität gelingt die Umsetzung bei Verwendung von Trichloräthylen. Bei Verwendung dieses Lösungsmittels werden die unerwünschten Nebenprodukte (2-Chlor-3-methyl-2-buten-1-al-acetale und 2,3-Dichlor-3-methyl-butan-1-al-acetale) nur in Ausbeuten von jeweils etwa 6% erhalten, während der Anteil ihrer Bildung bei Verwendung der übrigen genannten Halogenkohlenwasserstoffe höher ist.

Eine Chlorierung der Acetale II mit $SO_2Cl_2$ ohne Mitverwendung eines Lösungsmittels gelingt nicht.

Das Lösungsmittel verwendet man im allgemeinen in Mengen von etwa 0,5 bis 2 Liter, vorzugsweise etwa 1 Liter pro Mol des Ausgangsacetals.

Zur Durchführung der erfindungsgemäßen Chlorierung geht man im allgemeinen so vor, daß man das Acetal in dem Lösungsmittel löst und zu der erhaltenen, auf die erfindungsgemäße Temperatur erwärmte Lösung, vorzugsweise in eine unter Rückfluß siedende Lösung, das Sulfurylchlorid langsam einträgt. Das Eintragen von $SO_2Cl_2$ kann nicht beliebig schnell erfolgen. Die besten Ergebnisse erzielt man, wenn man etwa 1 Mol $SO_2Cl_2$ innerhalb von 1 bis 2 Stunden in die Lösung von 1 Mol des Ausgangsacetals einträgt. Hierbei erfolgt das Eintragen des $SO_2Cl_2$ etwa in dem Maße, wie das $SO_2Cl_2$ im Reaktionsgemisch verbraucht wird, d. h. wie die Chlorierung fortschreitet.

Das Sulfurylchlorid verwendet man erfindungsgemäß in etwa molaren Mengen wie das Ausgangsacetal, d. h. in Mengen von etwa 1 Mol bis 1,2 Mol pro Mol Acetal.

Die Umsetzung wird im allgemeinen unter Normaldruck durchgeführt.

Die Aufarbeitung des Reaktionsgemisches erfolgt auf übliche Weise durch Abdestillieren des Lösungsmittels und gegebenenfalls anschließendes Fraktionieren.

Soll das Verfahrensprodukt zur Herstellung der für Carotinoidsynthesen begehrten trans-3-Methyl-2-buten-1,4-dial-1-monoacetale verwendet werden, so kann man das Rohmaterial ohne weitere Reinigung einsetzen, da von den gebildeten Chloriden nur das 4-Chlor-Derivat zu dem entsprechenden Aldehyd oxidiert werden kann.

Sollen die trans-3-Methyl-2-buten-1,4-dial-1-monoacetale gleich für die Durchführung von Wittig-Reaktionen weiterverwendet werden, so können gleich die bei der Oxidation anfallenden Lösungen, beispielsweise die bei der Oxidation mit Dimethylsulfoxid (DMSO) anfallenden Lösungen ohne weitere Reinigung mit Phosphoniumsalzen umgesetzt werden.

Zur Herstellung der begehrten trans-3-Methyl-2-buten-1,4-dial-1-monoacetale können die Chloride der Formel I unter milden Reaktionsbedingungen oxidiert werden.

Eine vorteilhafte Methode zur Oxidation der Chloride der Formel I unter milden Reaktionsbedingungen ist beispielsweise die Oxidation von I mit Dimethylsulfoxid in Gegenwart von Basen wie Alkalicarbonaten, Alkalibicarbonaten, Erdalkalicarbonaten oder Erdalkalibicarbonaten. Daß diese Umsetzung mit guten Ausbeuten vorgenommen werden kann, war überraschend, da es im Fieser und Fieser »Reagents for Organic Synthesis«, John Wiley and Sons, Inc., New York 1967, Seite 303 heißt, daß primäre Chloride bei der Oxidation mit Dimethylsulfoxid nur sehr schlechte Ausbeuten ergäben und daß man deshalb die Chloride zunächst mit Silbertosylat in die Tosylate überführen müßte (vgl. auch Tetrahedron Letters No. 11 [1974] S. 917 f).

Zur Oxidation der Chloride der Formel I mit Dimethylsulfoxid erhitzt man im allgemeinen das Chlorid oder aber das bei der Chlorierung mit Sulfurylchlorid gemäß Verfahrensschritt A erhaltene Chloridgemisch in Gegenwart einer oder mehrerer der oben genannten Carbonate oder Hydrogencarbonate mit mindestens 1 Mol DMSO in einem inerten Lösungsmittel oder aber ohne inertes Lösungsmittel mit mindestens der 5fach molaren Menge an DMSO auf Temperaturen von etwa 50 bis 150°C, vorzugsweise 60 bis 120°C. Die Reaktionszeit beträgt im allgemeinen etwa 0,5 bis 2, vorzugsweise 1 bis 1,5 Stunden. Als inerte Lösungsmittel können beispielsweise Dimethylformamid oder Dimethylacetamid verwendet werden.

Mit Vorteil arbeitet man in überschüssigem Dimethylsulfoxid. Das Lösungsmittel verwendet man vorteilhaft in der etwa 5- bis 100fachen Gewichtsmenge der Ausgangsverbindung.

Verwendet man DMSO selbst als Lösungsmittel, so verwendet man dieses insgesamt in Mengen von 5 bis 20, vorzugsweise 5 bis 10 Mol pro Mol Chlorid der Formel I.

Die Basen verwendet man im allgemeinen in Mengen von 1 bis 10 Mol, vorzugsweise 2 bis 4 Mol pro Mol Chlorid.

Die Oxidation der Chloride der Formel I kann jedoch auch mit anderen bekannten milden Oxidationsmitteln vorgenommen werden. Genannt seien beispielsweise die Natriumsalze aliphatischer Nitroverbindungen wie 2-Nitro-propan und Nitrocyclohexan, Aminoxide, wie das Trimethylamin-

oxid und das N-Methyl-morpholinoxid sowie Hexamethylentetramin in der sogenannten Sommeletschen Reaktion.

Die Oxidation mit den Natriumsalzen der aliphatischen Nitroverbindungen kann man beispielsweise so durchführen, daß man je 1 Mol des Allylchlorids der Formel I, der Nitroverbindung und eines Natriumalkoholats oder von Natriumhydroxid in einem niederen aliphatischen Alkohol oder auch in Lösungsmitteln wie Dimethylformamid bei erhöhter Temperatur umsetzt. Da die Chlormethylgruppe sterisch etwas gehindert ist, empfiehlt es sich, dem Reaktionsgemisch geringe Mengen eines Hilfsnucleophils, wie KJ, zuzusetzen. Die Aufarbeitung erfolgt in üblicher Weise durch Gießen des Reaktionsgemisches auf Eiswasser, Extraktion und Destillation.

Auch die Oxidation mit Aminoxiden kann beispielsweise durch mehrstündiges Erhitzen des Allylchlorids I mit dem Aminoxid in Dimethylformamid und übliche Aufarbeitung erfolgen.

Die Oxidation mit Hexamethylentetramin beruht auf der Tatsache, daß sich die Allylchloride I unter Bildung quartärer Ammoniumsalze an Hexamethylentetramin (1 : 1 Mol) anlagern, welche sich beim Erhitzen mit Wasser leicht unter Bildung von dem Aldehyd I und Methylamin, neben Formaldehyd und Ammoniak hydrolysieren lassen. Hierbei ist es nicht unbedingt erforderlich, die quartären Verbindungen gesondert herzustellen. Beispielsweise kann man das Allylchlorid I durch Erhitzen mit Hexamethylentetramin in Dimethylformamid, dem etwas Wasser zugesetzt wurde, als Lösungsmittel in den Aldehyd der Formel I überführen.

Die Oxidation der Chloride der Formel I zu den Aldehyden der Formel I wird durch Beispiel 2A bis 2D beschrieben.

Bezüglich näherer Details über die Überführung von Chlormethylgruppen in Aldehydgruppen verweisen wir auf Methodicum Chimicum, Band 5, Georg Thieme Verlag, Stuttgart 1975, Seiten 290—293 oder Houben-Weyl, Methoden der Organischen Chemie, 4. Auflage, Band 7/1, Seiten 193—204.

Mit Hilfe des erfindungsgemäßen Verfahrens können die 6-Ring-Acetale des trans-4-Chlor-3-methyl-2-buten-1-al und hieraus die für Carotinoidsynthesen begehrten trans-3-Methyl-2-buten-1,4-dial-1-monoacetale auf einfache Weise mit guten Ausbeuten hergestellt werden.

## Beispiel 1

17 kg (100 Mol) 3-Methyl-2-buten-1-al-(2',2'-dimethyl-propylen)-acetal wurden in 120 Litern Trichloräthylen gelöst und die Lösung zum Sieden erhitzt. Zu der unter Rückfluß siedenden Lösung (87° C) wurden innerhalb von 90 Minuten 9 Liter (111 Mol) Sulfurylchlorid zugefügt und das Reaktionsgemisch noch 1 Stunde bei Siedetemperatur gehalten. Nach dem Abkühlen auf 20° C wurden zu dem Reaktionsgemisch 50 Liter Wasser und 10 kg $Na_2CO_3$ zugefügt, das ganze gerührt und anschließend eine Phasentrennung vorgenommen und die organische Phase eingeengt. Eine gaschromatographische Untersuchung ergab, daß das Ausgangsmaterial vollständig umgesetzt war. Die Ausbeute an 4-Chlor-3-methyl-2-buten-1-al-(2',2'-dimethyl-propylen)-acetal betrug 85% im Gemisch mit 6% des entsprechenden 2-Chlor-3-methyl-3-buten-1-al-acetals und 6% des entsprechenden 2,3-Dichlor-butan-1-al-acetals. Destillative Aufarbeitung ergab 15,6 kg entsprechend einer Ausbeute von 76% des 4-Chlor-Acetals.

## Beispiel 2

### A. Oxidation mit DMSO/$Na_2CO_3$

20 g des gemäß Beispiel 1 erhaltenen Gemisches wurden in 80 g Dimethylsulfoxid (DMSO) gelöst, die Lösung mit 20 g fein pulverisiertem $Na_2CO_3$ versetzt und unter Rühren auf 120° C erhitzt. Nach 2 1/2 Stunden Reaktionszeit waren 98% des 4-Chlor-3-methyl-2-buten-1-al-(2',2'-dimethyl-propylen)-acetals umgesetzt. Die im Gemisch enthaltenen Acetale von 2-Chlor-3-methyl-3-buten-1-al und 2,3-Dichlor-butan-1-al blieben unverändert. Eine gaschromatographische Untersuchung der Reaktionslösung ergab eine Ausbeute an trans-3-Methyl-2-buten-1,4-dial-1-(2',2'-dimethyl-propylen)-acetal von 73%, bezogen auf umgesetztes Chlorid.

Zur Aufarbeitung wurde das Reaktionsgemisch auf Wasser gegossen, mit Äther extrahiert und das Reaktionsprodukt fraktioniert destilliert. Ausbeute 67%. Bei Verwendung für Wittig-Reaktionen kann das erhaltene trans-3-Methyl-2-buten-1,4-dial-1-(2',2'-dimethyl-propylen)-acetal aber auch ohne weitere Reinigung gleich in der DMSO-Lösung mit Phosphoniumsalzen umgesetzt werden.

### B. Oxidation mit 2-Nitro-propan

10 g des gemäß Beispiel 1 erhaltenen destillierten Allylchlorids wurden zusammen mit 6 ml 2-Nitro-propan und 1 g KJ in 80 ml Dimethylformamid (DMF) gelöst. Die Lösung wurde auf 40° C

erwärmt und innerhalb von 60 Minuten tropfenweise mit 4,8 g einer 50%igen Natronlauge versetzt. Anschließend wurde das Reaktionsgemisch auf 20° C abgekühlt, noch eine Stunde unter Rühren stehenlassen und auf Eis/Wasser geschüttet. Durch Extraktion mit Toluol und anschließende Destillation erhielt man 6,2 g (entsprechend 69% der Theorie) trans-3-Methyl-2-buten-1,4-dial-1-(2',2'-di-methyl-propylen)-acetal vom Siedepunkt Kp = 71° C bei 0,1 mbar.

C. Oxidation mit Trimethylaminoxid

10 g des gemäß Beispiel 1 erhaltenen destillierten Allylchlorids wurden zusammen mit 7 g wasserfreiem Trimethylaminoxid in 60 ml Dimethylformamid gelöst. Die Lösung wurde 3 Stunden bei 60° C gerührt, dann abgekühlt und auf Wasser gegossen. Anschließende Extraktion mit Toluol und Destillation des Rohprodukts ergab 6,4 g (entsprechend 71% der Theorie) des trans-3-Methyl-2-buten-1,4-dial-1-(2',2'-dimethyl-propylen)-acetals.

D. Oxidation mit Hexamethylentetramin
(Sommelet-Reaktion)

10 g des gemäß Beispiel 1 erhaltenen destillierten Allylchlorids, 10 g Urotropin und 1 g KJ wurden in einer Mischung aus 100 ml DMF und 5 ml Wasser gelöst. Die Lösung wurde 1 Stunde lang auf 80° C erhitzt, dann abgekühlt und analog B. aufgearbeitet. Man erhielt 4,9 g (entsprechend 54% der Theorie) an trans-3-Methyl-2-buten-dialdehyd-1-(2',2'-dimethyl-propylen)-acetal.

Beispiel 3

17 g (0,1 Mol) 3-Methyl-2-buten-1-al-(2',2'-dimethyl-propylen)-acetal wurden in 100 ml Trichloräthylen gelöst und bei der in der folgenden Tabelle angegebenen Temperatur innerhalb von 1 Stunde mit 14,8 g (0,11 Mol) $SO_2Cl_2$ versetzt. Die jeweils erzielten Ausbeuten an 4-Chlor-3-methyl-2-buten-1-al-(2',2'-dimethyl-propylen)-acetal (A) bzw. 2-Chlor-3-methyl-3-buten-1-al-(2',2'-dimethyl-propylen)-acetal (B) und 2,3-Dichlor-butan-1-al-(2',2'-dimethyl-propylen)-acetal (C) sind in der Tabelle zusammengestellt.

| Solvens | Temperatur | Ausbeute %A | %B | %C |
|---|---|---|---|---|
| Trichloräthylen | 0°C | 0 | 0 | 100 |
| Trichloräthylen | 60°C | 35 | 25 | 35 |
| Trichloräthylen | 87°C (Rückfluß bei Normalbed.) | 85 | 6 | 6 |

Beispiele 4 bis 7 und Vergleichsbeispiele 8 bis 11

17 g (0,1 Mol) 3-Methyl-2-buten-1-al-(2',2'-dimethyl-propylen)-acetal wurden in jeweils 100 ml des in der Tabelle angegebenen Lösungsmittels gelöst und die Lösung zum Sieden erhitzt. Zu der unter Rückfluß siedenden Lösung wurden innerhalb von 1 Stunde 14,8 g (0,11 Mol) $SO_2Cl_2$ zugegeben. Die jeweils erzielten Ausbeuten an A, B und C (Erklärung s. Beispiel 3) sind in der folgenden Tabelle zusammengestellt.

| Beispiel | Solvens | Ausbeute % A | % B | % C |
|---|---|---|---|---|
| 4 | $CHCl_3$ | 43 | 37 | 0 |
| 5 | $C_2Cl_4$ | 65 | 20 | 0 |
| 6 | 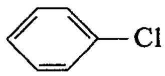 | 72 | 22 | 0 |
| 7 | $CH_2Cl_2$ | 41 | 34 | 14 |
| 8 | $C_7H_{16}$ | 33 | 23 | 28 |
| 9 | Petroläther (Kp = 60°C) | 0 | 0 | 100 |
| 10 | Cyclohexan | 0 | 0 | 100 |
| 11 | Petroläther (Kp = 90°C) | 0 | 0 | 100 |

**Patentansprüche**

1. Verfahren zur Herstellung von Acetalen der allgemeinen Formel I

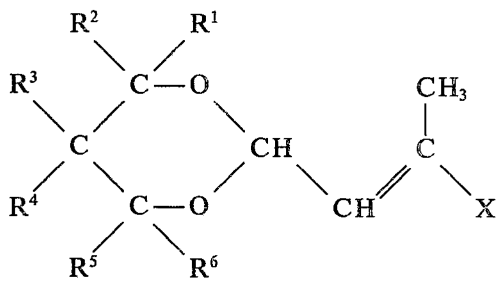

(I)

in der $R^1$ bis $R^6$ für $-H$, $-CH_3$ oder $-C_2H_5$, vorzugsweise $-H$ oder $-CH_3$ stehen, wobei vorzugsweise nur 1 bis 4 der Reste $R^1$ bis $R^6$ für $-CH_3$ und die restlichen für $-H$ stehen, und X für $-CH_2Cl$ oder

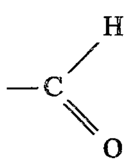

steht, dadurch gekennzeichnet, daß man

A) in die Lösung des entsprechenden Acetals von 3-Methyl-2-buten-1-al der Formel II

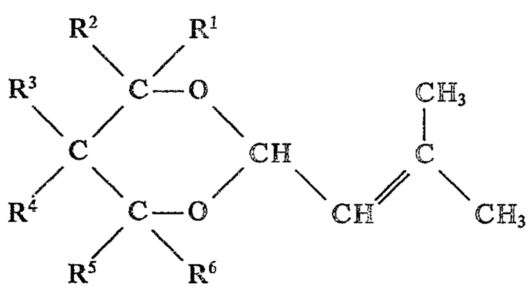

(II)

in einem Halogenkohlenwasserstoff vom Siedepunkt 50 bis 120°C bei Temperaturen von 50 bis 120°C molare bis leicht überschüssig molare Mengen von Sulfurylchlorid langsam und in dem Maße wie das Sulfurylchlorid im Reaktionsgemisch verbraucht wird, einträgt, und

B) für den Fall, daß X für

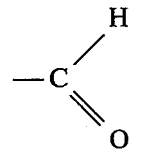

steht, das erhaltene 4-Chlor-3-methyl-but-2-en-1-al-acetal unter milden Reaktionsbedingungen oxidiert.

2. Verfahren gemäß Anspruch 1, dadurch gekennzeichnet, daß man im Reaktionsschritt A das Sulfurylchlorid in eine siedende Lösung des entsprechenden Acetals der Formel II in Trichloräthylen einträgt.

3. Verfahren gemäß Anspruch 1, dadurch gekennzeichnet, daß man im Reaktionsschritt A etwa 1,1 Mol Sulfurylchlorid innerhalb von 1 bis 2 Stunden in eine Lösung von etwa 1 Mol des Acetals II einträgt.

4. Verfahren gemäß Anspruch 1, dadurch gekennzeichnet, daß man im Reaktionsschritt B das 4-Chlor-3-methyl-but-2-en-1-al-acetal mit Dimethylsulfoxid in Gegenwart eines Alkalicarbonats, Alkalihydrogencarbonats, Erdalkalicarbonats oder Erdalkalihydrogencarbonats oxydiert.

**Claims**

1. A process for the preparation of an acetal of the general formula I

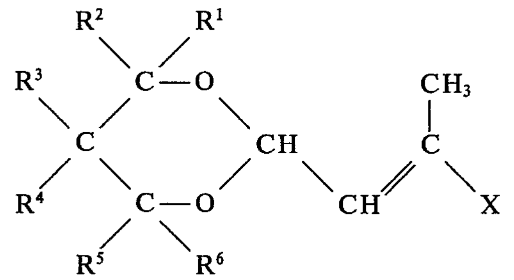

(I)

where $R^1$ to $R^6$ are $-H$, $-CH_3$ or $-C_2H_5$, preferably $-H$ or $-CH_3$, but preferably only from 1 to 4 of the radicals $R^1$ to $R^6$ are $-CH_3$ and the remainder are $-H$, and X is $-CH_2Cl$ or

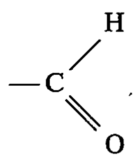

characterized in that

A) a molar amount, or slight excess, of sulfuryl chloride is introduced slowly into a solution of the corresponding acetal of 3-methyl-but-2-en-1-al of the formula II

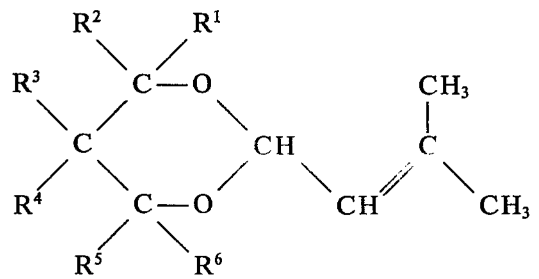

(II)

in a halohydrocarbon of boiling point 50 – 120°C, at 50 – 120°C, at the rate at which the sulfuryl chloride is consumed in the reaction mixture, and

B) where X is

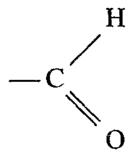

the resulting 4-chloro-3-methyl-but-2-en-1-al-acetal is oxidized under mild reaction conditions.

2. A process as claimed in claim 1, characterized in that, in reaction step A, the sulfuryl chloride is introduced into a boiling solution of the corresponding acetal of the formula II in trichloroethylene.

3. A process as claimed in claim 1, characterized in that, in reaction step A, about 1.1 moles of sulfuryl chloride are introduced, in the course of from 1 to 2 hours, into a solution of about 1 mole of the acetal II.

4. A process as claimed in claim 1, characterized in that, in reaction step B, 4-chloro-3-methyl-but-2-en-1-al-acetal is oxidized with dimethylsulfoxide in the presence of an alkali metal carbonate, alkali metal bicarbonate, alkaline earth metal carbonate or alkaline earth metal bicarbonate.

**Revendications**

1. Procédé de préparation d'acétals de la formule générale I

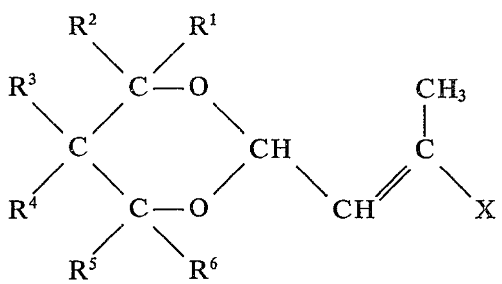

(I)

dans laquelle $R^1$ à $R^6$ désignent chacun un atome d'hydrogène ou un radical $-CH_3$ ou $-C_2H_5$ et de préférence un atome d'hydrogène ou un radical $-CH_3$, un à quatre des restes $R^1$ à $R^6$ désignant avantageusement un radical $-CH_3$ et les autres un atome d'hydrogène, et X représente un groupe $-CH_2Cl$ ou

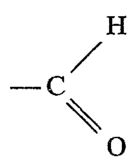

caractérisé en ce que:

A) à des températures de 50 à 120°C, on ajoute lentement et au fur et à mesure de sa consommation une proportion molaire ou un léger excès par rapport à la proportion molaire de chlorure de sulfuryle à la solution de l'acétal approprié du méthyl-3 butène-2 al-1 de la formule II

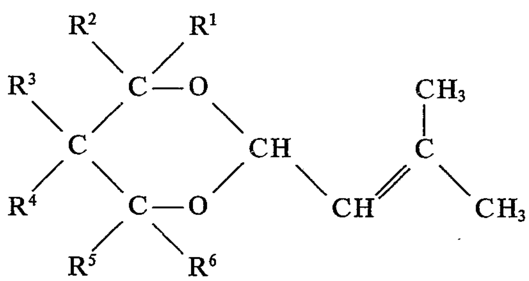

(II)

dans un hydrocarbure halogéné avec un point d'ébullition entre 50 et 120°C, et

B) dans le cas où X =

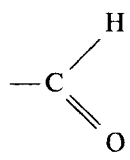

on oxyde l'acétal de chloro-4 méthyl-3 butène-2 al-1 obtenu dans des conditions réactionnelles modérées

2. Procédé suivant la revendication 1, caractérisé en ce que, dans le stade opératoire A, le chlorure de sulfuryle est introduit dans une solution bouillante de l'acétal approprié de la formule II dans le trichlor-éthylène.

3. Procédé suivant la revendication 1, caractérisé en ce que, dans le stade opératoire A, on ajoute environ 1,1 mole de chlorure de sulfuryle en l'espace de 1 à 2 heures à la solution d'environ 1 mole de l'acétal II.

4. Procédé suivant la revendication 1, caractérisé en ce que, dans le stade opératoire B, l'oxydation de l'acétal de chloro-4 méthyl-3 butène-2 al-1 est réalisée par le sulfoxyde de diméthyle en présence d'un carbonate ou hydrogéno-carbonate de métal alcalin ou d'un carbonate ou hydrogéno-carbonate de métal alcalino-terreux.